# EUROPEAN PATENT APPLICATION

(11) **EP 4 803 620 A1**
(43) Date of publication of application: **09.09.2026**
(21) Application number: 24885646.0
(22) Date of filing: 28.10.2024
(51) Int. Cl.: C12N 9/16, C12N 1/15, C12N 1/19, C12N 1/21, C12N 5/10, C12N 15/09, C12N 15/55, C12N 15/63, C12N 15/113

(54) **NOVEL MINIATURE CRISPR-CAS12**

(30) Priority: 30.10.2023 JP 2023185496
(71) Applicant: Ishino, Yoshizumi, Takatsuki-shi, Osaka 569-0814 (JP); Japan Software Management Co., Ltd., Yokohama-shi Kanagawa 221-0056 (JP)
(72) Inventor: NUMATA, Tomoyuki, Fukuoka-shi, Fukuoka 819-0385 (JP); MATSUMOTO, Shunsuke, Fukuoka-shi, Fukuoka 819-0385 (JP); ISHINO, Sonoko, Takatsuki-shi, Osaka 569-0814 (JP); TANAKA, Aoi, Fukuoka-shi, Fukuoka 819-0385 (JP); KIKKO, Osamu, Fukuoka-shi, Fukuoka 819-0385 (JP); NASU, Hisanori, Yokohama-shi, Kanagawa 221-0056 (JP); KOUYAMA, Haruka, Yokohama-shi, Kanagawa 221-0056 (JP); YOKOYAMA, Syoya, Yokohama-shi, Kanagawa 221-0056 (JP); ISHINO, Yoshizumi, Takatsuki-shi, Osaka 569-0814 (JP)
(74) Representative: Hoffmann Eitle
(86) International application number: PCT/JP2024/038286
(87) International publication number: WO 2025/094873

(57) **Abstract**

The present invention identifies novel CRISPR-Cas and provides a Cas protein derived therefrom.

A protein selected from the group consisting of:
(a) a protein comprising the amino acid sequence set forth in SEQ ID NO: 3;
(b) a protein comprising an amino acid sequence having at least 62% and less than 100% sequence identity to the amino acid sequence set forth in SEQ ID NO: 3, the protein being capable of forming a complex with a crRNA and binding to DNA having a sequence complementary to a spacer sequence in the crRNA; and
(c) a protein comprising an amino acid sequence in which 1 to 20 amino acids in the amino acid sequence set forth in SEQ ID NO: 3 are mutated, the protein being capable of forming a complex with a crRNA and binding to DNA having a sequence complementary to a spacer sequence in the crRNA.

Also provided are nucleic acids encoding the protein, recombinant vectors, and transformed cells. Kits and methods for genome editing are further provided.

## Description

### Technical Field

The present invention relates to a novel miniature CRISPR-Cas12 that serves as a tool for genetic engineering, including genome editing.

### Background Art

CRISPR (clustered regularly interspaced short palindromic repeats) refers to DNA sequences consisting of short repeat units, each typically several tens of base pairs in length, arranged at regular intervals within the genomes of prokaryotic organisms such as bacteria and archaea, and is known to function as an adaptive immune system in prokaryotes (Non-Patent Literature 1). Genes encoding CRISPR-associated (Cas) proteins, including nucleases and helicases, are located in the vicinity of CRISPR loci. A typical CRISPR locus comprises three elements: a *cas* gene cluster, a leader sequence, and repeat-spacer arrays. CRISPR-Cas systems are highly diverse and are classified into Class 1 and Class 2 according to the composition and action mechanism.
mechanisms of the Cas proteins. Class 1 includes Types I, III, and IV, whereas Class 2 includes Types II, V, and VI. Furthermore, each type is further subdivided into multiple subtypes (Non-Patent Literature 2).

CRISPR-Cas systems function by forming a complex between a CRISPR RNA (crRNA), transcribed from the spacer sequences within the CRISPR array, and a Cas nuclease. The crRNA guides the Cas nuclease to a DNA sequence complementary to the crRNA, where the nuclease introduces double-stranded DNA breaks. Based on this property, practical genome editing technologies have been developed and widely adopted worldwide (Non-Patent Literature 3). The most widely used system employs Cas9 derived from *Streptococcus pyogenes*, which belongs to Type II CRISPR-Cas systems. Subsequently, CRISPR-Cas systems derived from *Francisella novicida* have also been utilized. The nuclease protein originally named Cpf1 is now referred to as Cas12a in accordance with CRISPR-Cas classification.

CRISPR-Cas systems have been applied not only as genome editing technologies, but also various applications have been investigated by exploiting the property that a target protein can be freely guided to a desired position on the genome. Such applications include transcriptional regulation of specific genes, site-specific epigenetic modification, site-specific imaging, and nucleic acid detection methods (Non-Patent Literature 4, 5).

### Prior Art Documents

### Non-Patent Literature

Non-Patent Literature 1: Barrangou R. et al., Science, Vol. 315, No. 6, pp. 1709-1712, 2007.
Non-Patent Literature 2: Makarova K. S. et al., Nature Reviews Microbiology, Vol. 18, No. 11, pp. 67-83, 2019.
Non-Patent Literature 3: Jinek M. et al., Science, Vol. 337, pp. 810-821, 2012.
Non-Patent Literature 4: Knott G. J. et al., Science, Vol. 361, No. 6405, pp. 866-869, 2018.
Non-Patent Literature 5: Mazhar A. et al., Nature Communications, 9(1):1911, 2018.

### Disclosure of the Invention

### Problem for Solution by the Invention

CRISPR-Cas9 constitutes a highly versatile core system for genome editing, and CRISPR-Cas12 has also recently attracted attention. However, these Cas proteins are relatively large in molecular size, which results in low efficiency of delivery into cells. Accordingly, in order to improve genome editing technologies, it is essential to identify novel Cas proteins with smaller molecular sizes. In addition, shorter crRNAs are advantageous for guiding Cas effectors with DNA cleavage activity to target DNA sites.

An object of the present invention is to provide a novel miniature Cas protein.

### Means for Solving the Problems

The present inventors have previously analyzed metagenomes derived from marine samples and identified a large number of CRISPR arrays. Furthermore, the inventors identified an open reading frame (ORF) located in the vicinity of a CRISPR array, which is presumed to have an amino acid sequence similar to RuvC, an active domain of Cas12. The ORF encodes a protein of approximately 500 amino acid residues, which is less than half the size of Cas9 or Cas12. With the aim of application to genome editing, the present inventors performed functional and structural analyses of a miniature Cas12 protein designated TD1.

TD1 was purified as an RNA-bound complex by co-expression with a CRISPR array in *Escherichia coli*. RNA-seq analysis revealed that the RNA bound to TD1 was a crRNA that had been transcribed from the CRISPR array and subsequently processed. In order to determine the PAM sequence required for TD1-mediated double-stranded target DNA cleavage, a plasmid library having random nucleotide sequences at the 5'-terminal side of the protospacer was constructed. By mixing the TD1-crRNA complex with the plasmid library and subjecting plasmids cleaved by TD1 to next-generation sequencing analysis, the PAM sequence of TD1 was determined. It was also confirmed that the target DNA cleavage activity of TD1 depends on conserved acidic residues within the predicted RuvC domain. A ternary complex of TD1, a crRNA, and target DNA was reconstituted, and its structure was determined at a resolution of 2.9 Å by single-particle analysis using cryo-electron microscopy. In order to elucidate the mechanism of target DNA cleavage by TD1, mutational analyses based on the structural information were performed.

The present invention has been completed based on these findings. The summary of the present invention is as follows.
(1) A protein selected from any one of the following (a), (b), or (c):
   (a) a protein comprising the amino acid sequence set forth in SEQ ID NO: 3;
   (b) a protein comprising an amino acid sequence having 62% or more and less than 100% identity to the amino acid sequence set forth in SEQ ID NO: 3, which forms a complex with a crRNA and is capable of binding to DNA having a sequence complementary to a spacer sequence in the crRNA;
   (c) a protein comprising an amino acid sequence in which 1 to 20 amino acids are mutated in the amino acid sequence set forth in SEQ ID NO: 3, which forms a complex with a crRNA and is capable of binding to DNA having a sequence complementary to a spacer sequence in the crRNA.
(2) The protein according to (1), which has nuclease activity.
(3) The protein according to (1), which does not have nuclease activity.
(4) A nucleic acid encoding the protein according to (1).
(5) An expression vector comprising DNA encoding the protein according to (1).
(6) A cell transformed with the expression vector according to (5).
(7) A method for producing a protein selected from the group consisting of (a), (b), or (c), comprising culturing the cell according to (6):
   (a) a protein comprising the amino acid sequence set forth in SEQ ID NO: 3;
   (b) a protein comprising an amino acid sequence having 62% or more and less than 100% identity to the amino acid sequence set forth in SEQ ID NO: 3, which forms a complex with a crRNA and is capable of binding to DNA having a sequence complementary to a spacer sequence in the crRNA;
   (c) a protein comprising an amino acid sequence in which 1 to 20 amino acids are mutated in the amino acid sequence set forth in SEQ ID NO: 3, which forms a complex with a crRNA and is capable of binding to DNA having a sequence complementary to a spacer sequence in the crRNA.
(8) A method for cleaving DNA having a sequence complementary to a spacer sequence in a crRNA, using the protein according to (1) and a crRNA.
(9) A method for genome editing, using the protein according to (1) and a crRNA.
(10) A kit for genome editing, comprising at least one selected from the group consisting of the protein according to (1), mRNA encoding the protein according to (1), and an expression vector comprising DNA encoding the protein according to (1).
(11) A crRNA having a repeat sequence comprising any one of the nucleotide sequences set forth in SEQ ID NOS: 4 to 8.
(12) The protein according to (1), wherein the protein consists of an amino acid sequence homologous to the amino acid sequence set forth in SEQ ID NO: 3, and wherein, in the amino acid sequence, acidic amino acids or basic amino acids at positions corresponding to Asp156, Arg191, and Arg193 in the amino acid sequence set forth in SEQ ID NO: 3 form hydrogen bonds with bases in a PAM sequence of target DNA.
(13) The crRNA according to (11), wherein the spacer forms an R-loop with target DNA.

### Effects of the Invention

According to the present invention, a novel CRISPR-Cas system and a method for producing the same are provided. The novel CRISPR-Cas belongs to Class 2, Type V in the currently known classification system, and functions as an effector involved in target DNA cleavage using a single peptide. Accordingly, a practical genome editing tool is provided. Furthermore, since the size of this effector peptide is markedly smaller than those currently used for genome editing, an easy-to-handle tool with higher delivery efficiency is provided.

The present specification incorporates the contents described in the specification and/or drawings of Japanese Patent Application No. 2023-185496 based on which the present patent application claims priority.

### Brief Description of the Drawings

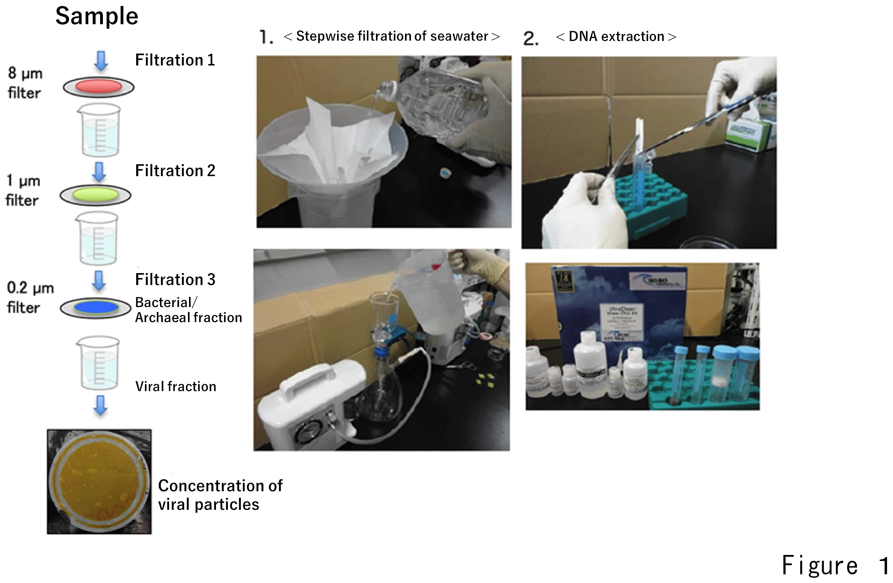

[Figure 1] Procedure for environmental metagenomic analysis. Seawater collected from a fixed sampling point in Sendai Bay was subjected to stepwise filtration, and DNA was prepared from microorganisms retained on a 0.22 µm filter and subjected to NGS sequencing analysis.

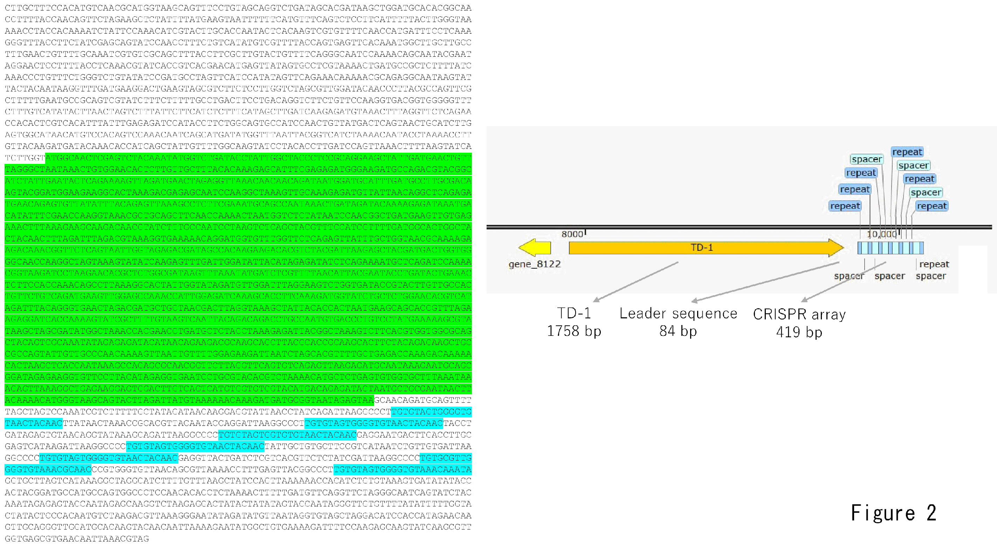

[Figure 2] Identification of a CRISPR-Cas region from metagenomic sequences. Sequence data obtained were assembled to generate sequence data as continuous as possible, and a database was constructed. As a result of searching this database for CRISPR-like repetitive sequences, a gene region as shown in the figure (the nucleotide sequence is shown in SEQ ID NO: 1) was identified. Arrows indicate regions translated into amino acid sequences and their directions of translation.

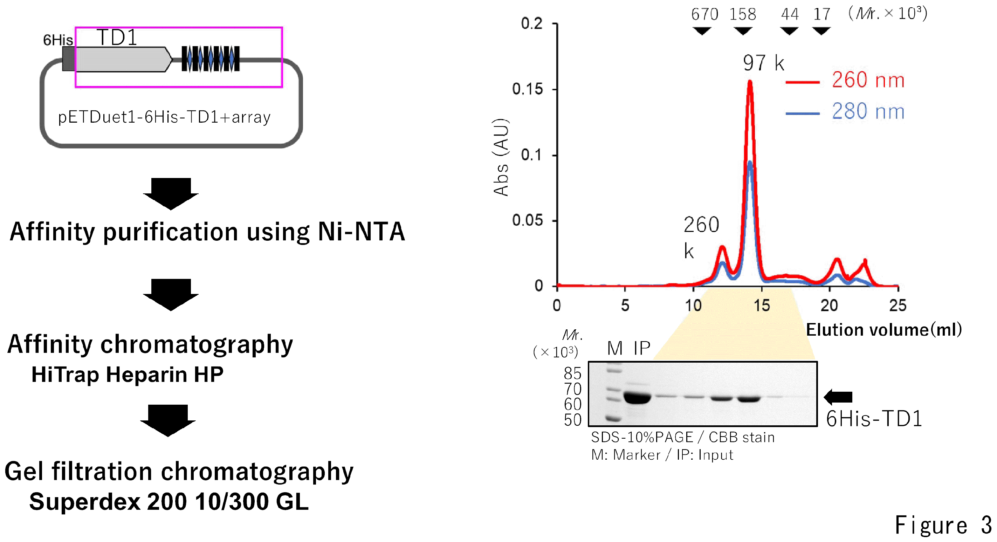

[Figure 3] Co-expression of TD1 and a surrounding region containing a CRISPR array. In order to identify crRNA that binds to the TD1 protein, RNA transcribed from the CRISPR region was assumed to be crRNA. TD1 and a downstream CRISPR region were inserted into an expression vector suitable for *E. col* cells, and the constructed plasmid was introduced into *E. coli* cells for co-expression of TD1 and a crRNA. Cell lysates of *E. coli* were subjected to Ni-NTA affinity chromatography via a His-tag added to TD1, followed by heparin affinity chromatography and gel filtration. As a result, TD1 was eluted as a single peak, and the peak fraction showed higher absorbance at A₂₆₀ than at A₂₈₀, indicating the presence of nucleic acids in the sample fraction.

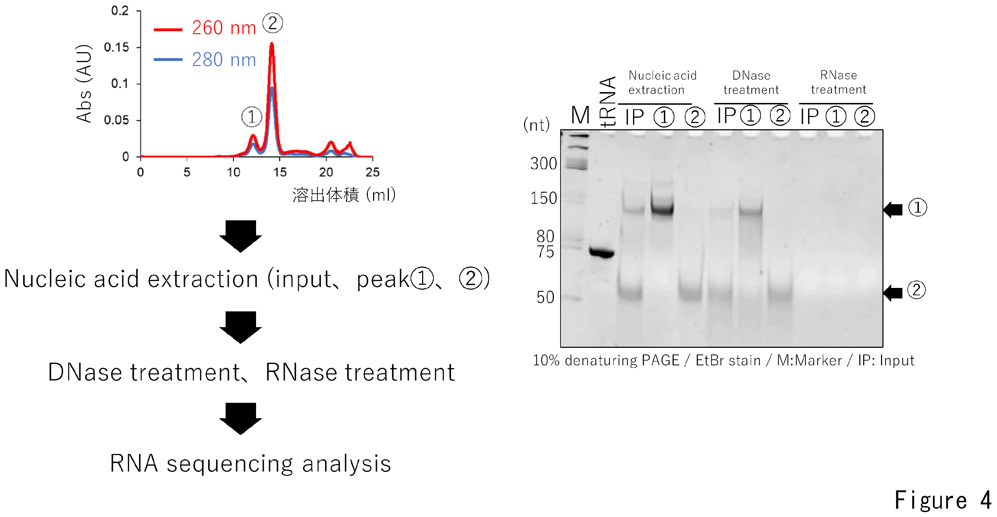

[Figure 4] Identification of nucleic acids bound to TD1. Nucleic acids were extracted from the peak fractions exhibiting absorbance at 260 nm and 280 nm as shown in Figure 3, and two specific bands were observed. Subsequent DNase and RNase treatments revealed that the bands disappeared only after RNase treatment, indicating that the nucleic acids contained together with the TD1 protein were RNA.

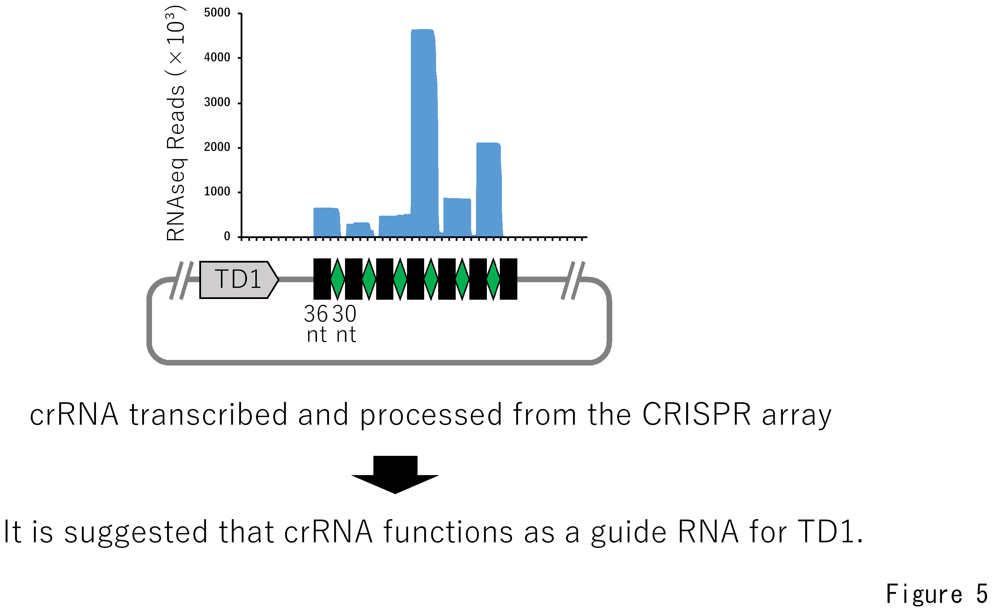

[Figure 5] Mapping of RNA sequencing analysis results onto the plasmid. The horizontal axis represents each position, and the vertical axis represents the number of RNA-seq reads. A high number of reads were observed at positions corresponding to the CRISPR array, spanning 60 nt, indicating crRNA that had been transcribed from and processed from the CRISPR array. These results suggest that crRNA functions as the guide RNA of TD1.

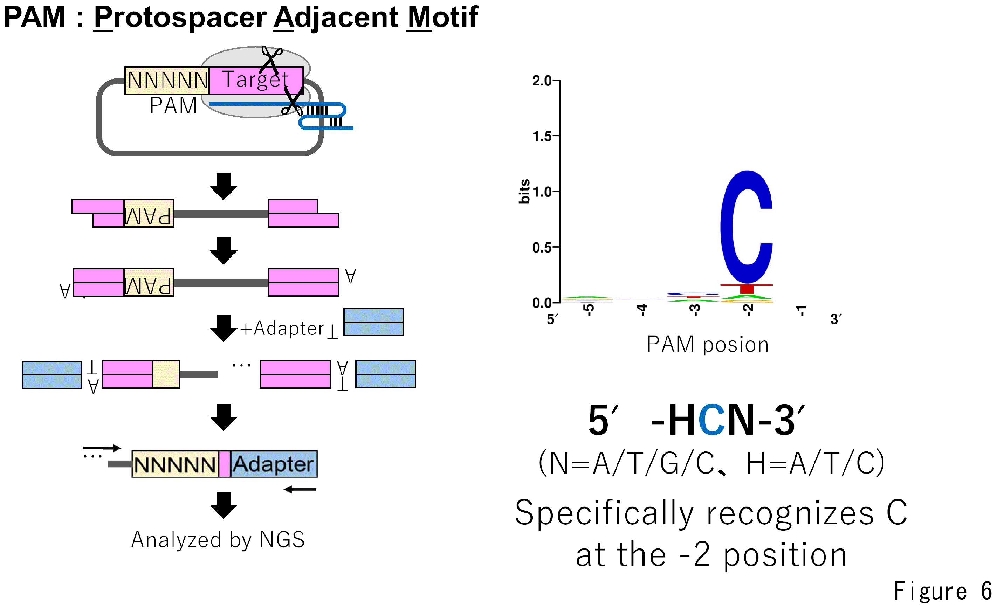

[Figure 6] PAM sequence recognized by TD1. The PAM sequence is required to distinguish self-genomic DNA from foreign nucleic acids. After target DNA cleavage of the PAM-library plasmid by TD1, the protruding end were filled, adapters were added, and analysis of preferred PAM for TD1 was performed by NGS. As a result, it was found that TD1 recognizes 5'-HCN-3' as a PAM sequence, in which the -2 position is specifically cytosine.

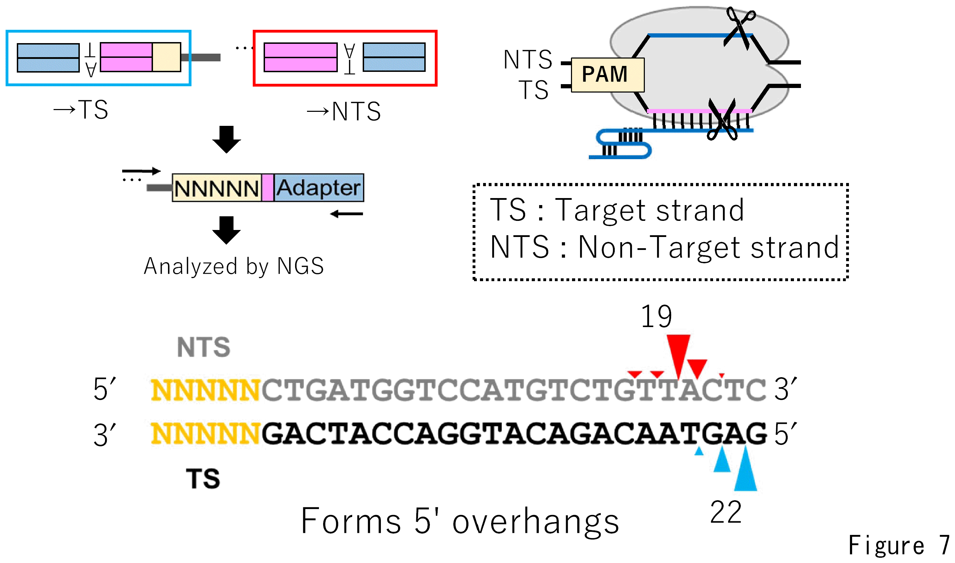

[Figure 7] Identification of cleavage positions in the target sequence by TD1. Based on the sequence analysis shown in Figure 6, the cleavage positions by TD1 were estimated. It was found that the non-target strand (NTS) is cleaved at a position 19 (20) nt downstream from the PAM, and the target strand (TS) is cleaved at a position 22 (21) nt downstream from the PAM, thereby forming 5' overhangs

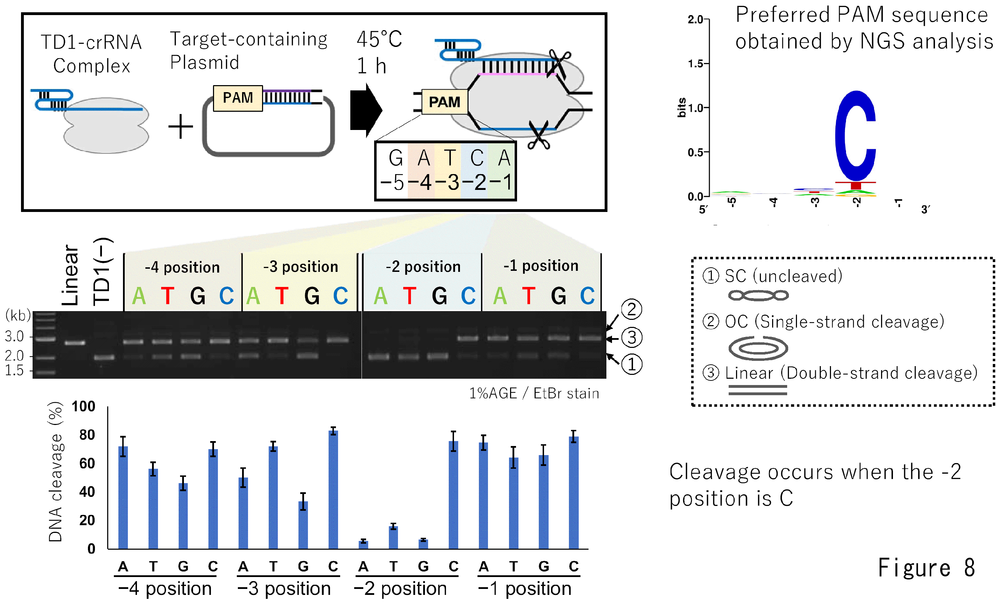

[Figure 8] Sequence specificity of the PAM. Using one of the recognized PAM sequences, 5'-GATCA-3', sequences were prepared in which the 1st, 2nd, 3rd, and 4th bases from the 3' end were individually substituted with each of the four nucleotides, and cleavage efficiencies were compared. The horizontal axis of the bar graph represents each nucleotide, and the vertical axis represents the proportion of DNA cleaved. No apparent differences were observed at the - 4 and -1 positions. At the -3 position, A, T, and C were preferred over G, whereas at the -2 position, cleavage occurred only when cytosine was present. These results indicate that TD1 specifically cleaves DNA at a PAM sequence having cytosine at the -2 position, which is consistent with the NGS results.

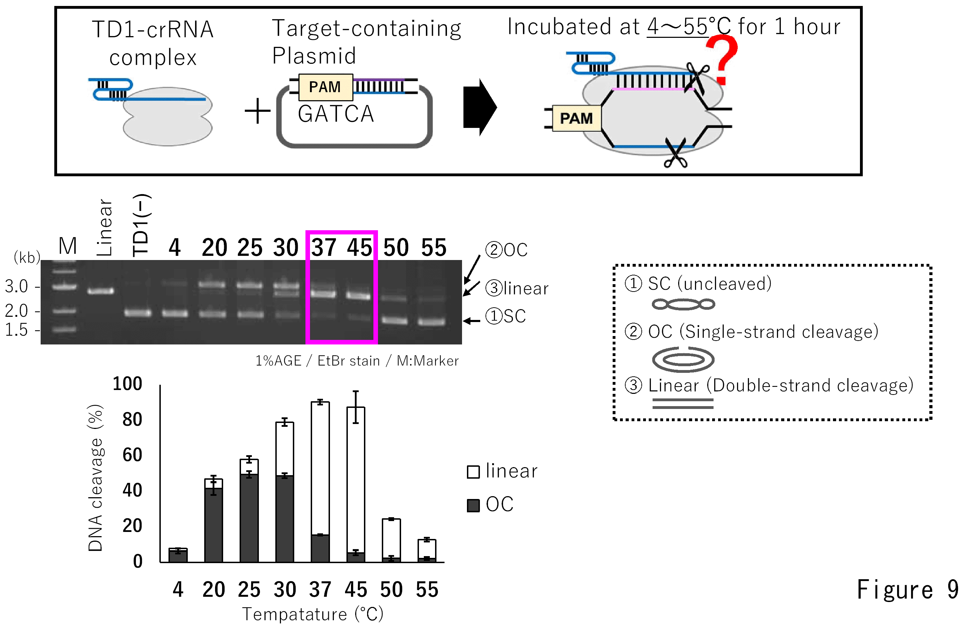

[Figure 9] Temperature at which TD1 cleaves DNA. The TD1-crRNA complex and a plasmid containing cytosine at the -2 position of the PAM were incubated at temperatures ranging from 4°C to 50°C. As a result, it was revealed that target DNA was markedly cleaved at 37-44°C, suggesting the potential for application in human cells.

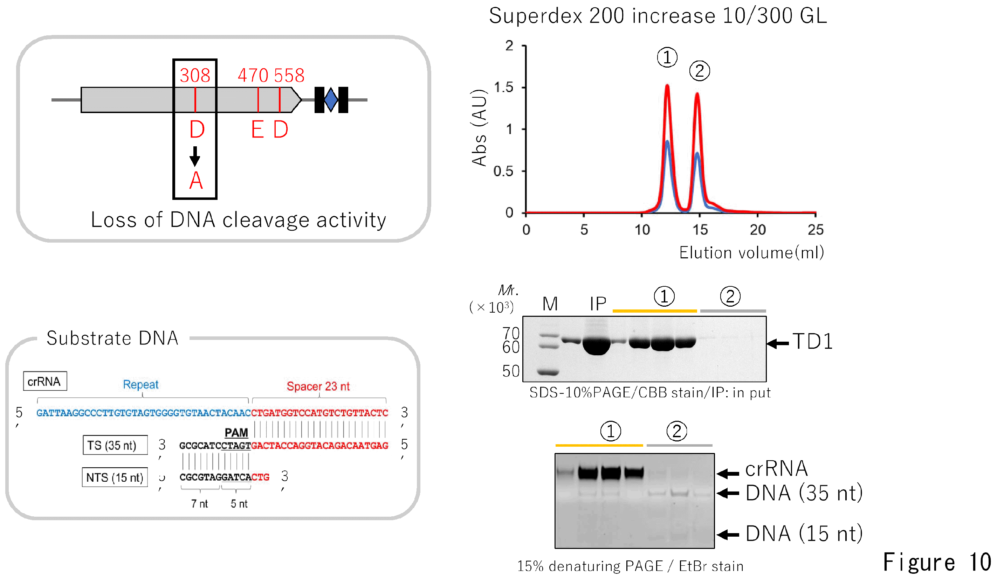

[Figure 10] Preparation of a TD1 mutant (a mutant in which Asp308 was substituted with alanine). A mutation was introduced into the DNA cleavage active site of TD1 to eliminate DNA cleavage activity, and the resulting TD1-crRNA complex was purified. The complex was mixed with target double-stranded DNA, incubated to reconstitute, and isolated by gel filtration chromatography. In the peak fraction [1], TD1, crRNA, and DNA were detected, and this fraction was subjected to structural analysis by cryo-electron microscopy.

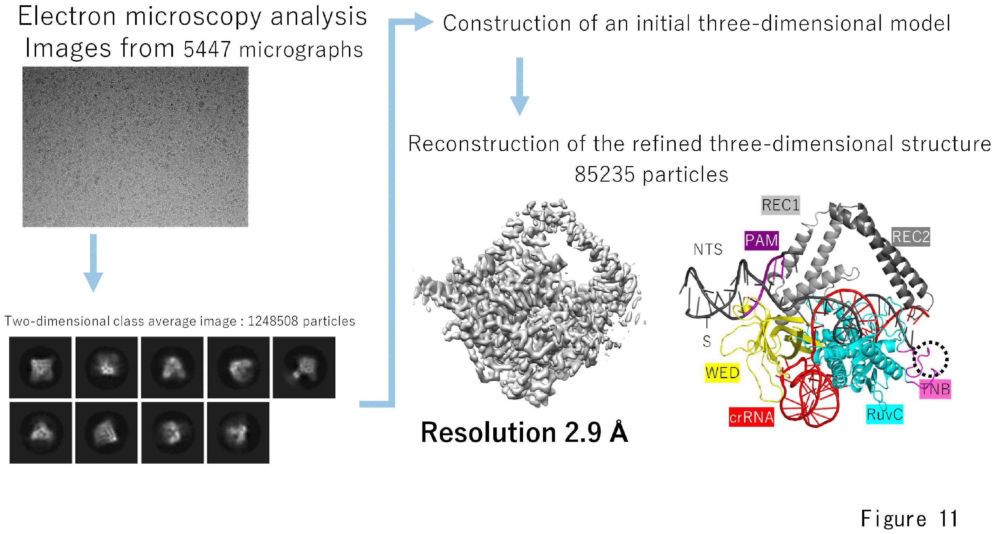

[Figure 11] Three-dimensional image of a TD1-crRNA complex lacking DNA cleavage activity and target DNA. The peak fraction [1] shown in Figure 10 was subjected to cryo-electron microscopy observation. From 5,447 electron micrographs obtained, 1,248,508 particles considered to be the target molecule were selected and classified based on structural similarity to generate two-dimensional averaged images. Based on these images, an initial three-dimensional structure was constructed, and a refined three-dimensional structure model was reconstructed using a dataset presumed to be TD1 as a reference. As a result, a map with a resolution of 2.9 Å was obtained.

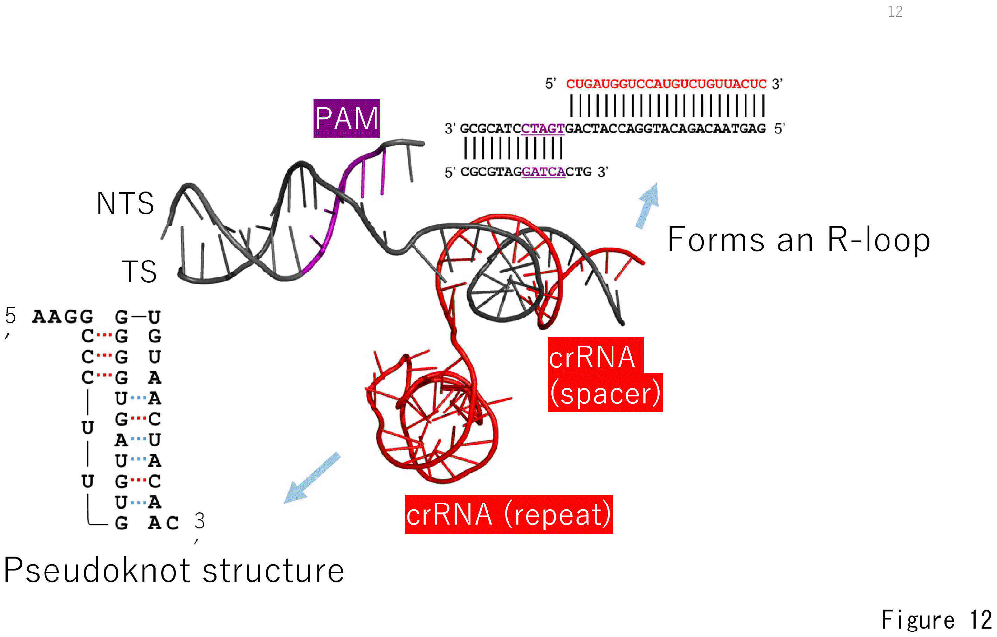

[Figure 12] Three-dimensional structure of crRNA. In the refined three-dimensional structure shown in Figure 11, the nucleic acid corresponding to the crRNA repeat was found to adopt a pseudoknot structure, and the spacer was found to form an R-loop with the target DNA.

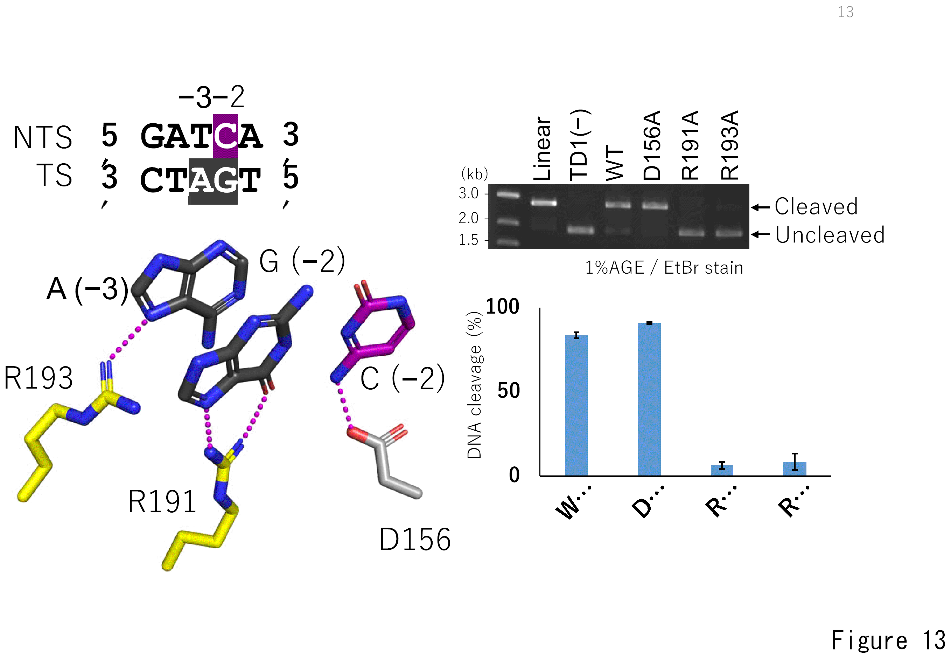

[Figure 13] Identification of amino acid residues capable of recognizing the PAM. It was revealed that cytosine at the -2 position of the non-target strand forms hydrogen bonds with Asp156, and that guanine at the -2 position and adenine at the -3 position of the target strand form hydrogen bonds with Arg191 and Arg193, respectively, indicating base-specific recognition by TD1. Functional and structural analyses revealed that TD1 is a Cas protein that functions as a monomer, and suggested the potential for application as a genome editing tool with a smaller molecular size than existing Cas proteins.

### Mode for Carrying Out the Invention

Hereinafter, modes for carrying out the present invention will be described.

The present invention provides any one of the following proteins (a), (b), or (c):
(a) a protein comprising the amino acid sequence of SEQ ID NO: 3;
(b) a protein comprising an amino acid sequence having 62% or more and less than 100% identity to the amino acid sequence of SEQ ID NO: 3, which is capable of forming a complex with crRNA and binding to DNA having a sequence complementary to the spacer sequence in the crRNA;
(c) a protein comprising an amino acid sequence in which 1 to 20 amino acids are mutated in the amino acid sequence of SEQ ID NO: 3, which is capable of forming a complex with crRNA and binding to DNA having a sequence complementary to the spacer sequence in the crRNA.

The amino acid sequence of SEQ ID NO: 3 is most closely related to ARI, which is a Cas protein discovered by the present inventors (Japanese Patent Application No. 2023-148437), and has 61.7% identity thereto. For calculation of sequence identity, for example, analysis software such as MAFFT can be used.

The protein comprising the amino acid sequence of SEQ ID NO: 3 (the protein of (a) above) preferably has nuclease activity. This activity includes the property that the protein forms a complex with crRNA produced by processing of pre-crRNA generated by transcription of a CRISPR region, and, depending on the sequence possessed by the crRNA, guides the polypeptide consisting of the amino acid sequence of SEQ ID NO: 3 to a DNA strand having a complementary sequence as a target and cleaves both strands of the DNA. However, the protein of the present invention does not necessarily have nuclease activity. The protein of the present invention can form a complex with crRNA and bind to DNA (target DNA) having a sequence complementary to the spacer sequence in the crRNA.

The crRNA forming a complex with the protein of the present invention preferably has a repeat sequence including a sequence obtained by removing four bases (GATT) on the 5' side from the CRISPR repeat sequence (double-underlined) in the sequence of SEQ ID NO: 1 (sequence of a gene region encoding a novel CRISPR-Cas). Sequences obtained by removing four bases (GATT) on the 5' side from the CRISPR repeat sequences in the sequence of SEQ ID NO: 1 are shown in SEQ ID NOs: 4 to 8. CRISPR repeat sequences are generally considered to be diverse, and the CRISPR repeat sequences present in the sequence of SEQ ID NO: 1 show differences of 1, 2, or 7 bases compared with the first CRISPR repeat sequence. These CRISPR repeat sequences having different bases may have equivalent functions. The length of the repeat sequence is preferably 23 to 55 bp, more preferably 24 to 38 bp, and even more preferably 29 to 30 bp.

The crRNA forming a complex with the protein of the present invention preferably has a spacer that forms an R-loop with the target DNA. The length of the spacer sequence is preferably 26 to 51 bp, and more preferably 30 to 45 bp.

For a Cas effector protein to act on target DNA, Cas and crRNA form a complex, Cas is guided to DNA having a sequence homologous to the spacer RNA portion of the crRNA, and Cas recognizes a PAM sequence present there, thereby exhibiting a function of cleaving double-stranded DNA. In structural analysis of the Cas-crRNA-DNA complex of the present invention, it was confirmed that the amino acid residues for PAM recognition by the Cas of the present invention were identified, and that the crRNA cleaves the double-stranded target DNA and forms a hybrid strand with one strand thereof (R-loop formation). The repeat sequence portion of the crRNA was a 36-mer of 5'-GATTAAGGCCCTTGTGTAGTGGGGTGTAACTACAAC-3'. However, in the obtained structure, the four bases (GATT) on the 5' side were not observed, suggesting that they were cleaved during analysis or that the structure was highly flexible. Therefore, it is considered that a 32-mer crRNA of 5'-AAGGCCCTTGTGTAGTGGGGTGTAACTACAAC-3' (SEQ ID NO: 4) is sufficient for the Cas-crRNA of the present invention to exert the intended function.

Binding of the complex to the target DNA can be directly confirmed by cleavage of the target DNA (when the protein of the present invention has nuclease activity), labeling of the target DNA (when the protein of the present invention lacks nuclease activity and is labeled), use of various molecular biological experimental methods depending on the labeling method, or by crystal structure analysis and the like. In the examples described later, based on three-dimensional model construction obtained from electron microscopic observation and cleavage activity of TD1 mutants, it was confirmed that Asp156, Arg191, and Arg193 in the protein (TD1) comprising the amino acid sequence of SEQ ID NO: 3 form hydrogen bonds with bases in the PAM sequence of the target DNA.

The protein of the present invention and crRNA form a complex, and the crRNA has a property of guiding the protein of the present invention onto a target DNA strand; however, tracrRNA required by Cas9, which is currently widely used, is not necessarily required. That is, the above properties can be exhibited with a simpler molecular composition. As the PAM (protospacer adjacent motif) sequence for distinguishing self DNA from target DNA, specific cleavage of a target sequence is possible if the sequence is 5'-HC-3' (H = A/T/C).

crRNA can be artificially synthesized in order for the protein of the present invention to cleave a target DNA strand or be guided to a site on the target DNA. In genome editing, crRNA is generally referred to as guide RNA, and a polypeptide that cleaves target DNA is often referred to as an effector. The guide RNA only needs to be able to form base pairs with the target DNA while in a complex with the effector, and its length is not particularly limited, but is typically about 100 nucleotides. To obtain the desired activity, the effector and the guide RNA may be prepared separately and mixed at the time of use, or they may be prepared as a complex and used in a reaction.

The protein of (b) above comprising an amino acid sequence having 62% or more and less than 100% identity with the amino acid sequence of SEQ ID NO: 3, forms a complex with crRNA, and is capable of binding to DNA having a sequence complementary to the spacer sequence in the crRNA.

The identity between the amino acid sequence of the protein of (b) above and the amino acid sequence of SEQ ID NO: 3 is 62% or more and less than 100%, and is preferably, for example, 65% or more, 70% or more, 75% or more, 80% or more, 85% or more, 90% or more, or 95% or more and less than 100%. For calculation of sequence identity, for example, analysis software such as MAFFT can be used.

The protein of (c) above comprising an amino acid sequence in which 1 to 20 amino acids are mutated in the amino acid sequence of SEQ ID NO: 3, forms a complex with crRNA, and is capable of binding to DNA having a sequence complementary to the spacer sequence in the crRNA.

The protein of (c) above may comprise an amino acid sequence in which one or more amino acids are deleted, substituted, or added in the amino acid sequence of SEQ ID NO: 3. The total number of amino acids deleted, substituted, or added is one or more, and the specific range is typically 1 to 20, 1 to 15, or 1 to 10, preferably 1 to 5, and more preferably 1 to 2 for deletions; similarly 1 to 20, 1 to 15, or 1 to 10, preferably 1 to 5, and more preferably 1 to 2 for substitutions; and 1 to 20, 1 to 15, or 1 to 10, preferably 1 to 5, and more preferably 1 to 2 for additions.

The proteins of (b) and (c) above comprise amino acid sequences homologous to the amino acid sequence of SEQ ID NO: 3, and in those amino acid sequences, acidic or basic amino acids at positions corresponding to Asp156, Arg191, and Arg193 in the amino acid sequence of SEQ ID NO: 3 are capable of forming hydrogen bonds with bases in the PAM sequence of the target DNA.

The protein of the present invention may be either a glycosylated protein or a nonglycosylated protein. The type and position of glycans added to the protein differ depending on the type of host cell used in protein production, and glycosylated proteins include proteins obtained using any host cell.

The present invention also provides a nucleic acid encoding any one of the proteins of (a), (b), or (c) above. The nucleic acid may be single-stranded DNA, single-stranded RNA (for example, mRNA), a single-stranded polynucleotide consisting of a mixture of DNA and RNA, double-stranded DNA, double-stranded RNA, a DNA-RNA hybrid polynucleotide, or a double-stranded polynucleotide consisting of two types of polynucleotides composed of a mixture of DNA and RNA. Using DNA encoding any one of the proteins of (a), (b), or (c) above, the corresponding protein can be produced. RNA (mRNA) encoding any one of the proteins of (a), (b), or (c) above can be used for genome editing.

The sequence of DNA encoding the protein comprising the amino acid sequence of SEQ ID NO: 3 is shown in SEQ ID NO: 2. DNA having the sequence of SEQ ID NO: 2 can be produced by known artificial gene synthesis methods. For example, a series of oligonucleotides having overlapping sequences are synthesized and annealed to form a double-stranded DNA fragment containing nicks in both strands. Repairing these nicks with DNA ligase yields an artificial gene having the desired sequence. RNA (mRNA) having the sequence of SEQ ID NO: 2 can be produced by preparing plasmid DNA encoding the sequence of SEQ ID NO: 2, amplifying template DNA by PCR, purifying it, and performing a transcription reaction using RNA polymerase.

DNA encoding the proteins of (b) and (c) above can be obtained by introducing mutations into DNA having the sequence of SEQ ID NO: 2 using known methods such as site-directed mutagenesis. Mutation introduction can be carried out using, for example, mutation introduction kits such as Mutan-K (manufactured by TAKARA), Mutan-G (manufactured by TAKARA), the LA PCR in vitro Mutagenesis series kits from TAKARA, or similar kits commercially available from various manufacturers based on the same principle. Alternatively, the DNA can also be obtained by chemically synthesizing the entire gene region using oligo DNA prepared to include mutations at desired positions.

The protein of the present invention can be produced, for example, by preparing a recombinant vector in which DNA encoding the protein of the present invention is inserted into an expression vector, transforming host cells with the recombinant vector, culturing the transformed cells, and purifying the protein produced by the transformed cells.

In preparing a recombinant vector, first, a DNA fragment of an appropriate length containing the coding region of a protein of interest is prepared. In the nucleotide sequence of the coding region of the protein, nucleotides may be substituted so as to provide codons optimal for expression in the host cell.

Next, the DNA fragment is inserted downstream of a promoter of an appropriate expression vector to prepare a recombinant vector. The DNA fragment needs to be incorporated into the vector so that its function is exerted. The vector may contain, in addition to the promoter, cis-elements such as enhancers, splicing signals, polyA addition signals, selection markers (for example, dihydrofolate reductase gene, ampicillin resistance gene, neomycin resistance gene), ribosome-binding sequences (SD sequences), and the like.

The expression vector is not particularly limited as long as it can autonomously replicate in a host cell, and examples include plasmid vectors, phage vectors, and viral vectors. Examples of plasmid vectors include plasmids derived from *E. coli* (for example, pRSET, pBR322, pBR325, pUC118, pUC119, pUC18, pUC19), plasmids derived from *Bacillus subtilis* (for example, pUB110, pTP5), and plasmids derived from yeast (for example, YEp13, YEp24, YCp50). Examples of phage vectors include λ phages (for example, Charon4A, Charon21A, EMBL3, EMBL4, λgt10, λgt11, λZAP). Examples of viral vectors include animal viruses such as retrovirus, vaccinia virus, adenovirus, and adeno-associated virus (AAV), and insect viruses such as baculovirus.

The recombinant vector, in which DNA encoding the protein of the present invention is inserted into an expression vector can also be used for genome editing.

Transformant cells capable of producing a protein of interest can be obtained by introducing the recombinant vector into an appropriate host cell.

As the host cell, any cell capable of expressing an inserted gene to produce a encoding protein of interest may be used, including prokaryotic cells, yeast, animal cells, insect cells, and plant cells. Animal individuals, plant individuals, and silkworm larvae may also be used.

When bacteria are used as host cells, bacteria belonging to genera such as *Escherichia* (for example, *E. coli*), *Bacillus* (for example, *B. subtilis*), *Pseudomonas* (for example, *P. putida*), and *Rhizobium* (for example, *R. meliloti*) can be used. Specifically, *E. coli* BL21, *E. coli* XL1-Blue, *E. coli* XL2-Blue, *E. coli* DH1, *E. coli* K12, *E. coli* JM109, *E. coli* HB101, and the like, and *B. subtilis* MI114, *B. subtilis* 207-21, and the like can be used as host cells. In this case, the promoter is not particularly limited as long as it can function in bacteria such as *E. coli,* and examples include promoters derived from *E. coli* or phages such as the *trp* promoter, *lac* promoter, *P*_{L} promoter, *P*_{R} promoter. Artificially designed and modified promoters such as the tac promoter, *lac*T7 promoter, and *let*I promoter can also be used.

Methods for introducing recombinant vectors into bacteria are not particularly limited as long as they allow introduction of DNA into bacteria, and examples include calcium ion methods and electroporation.

When yeast is used as a host cell, *Saccharomyces cerevisiae*, *Schizosaccharomyces pombe*, *Pichia pastoris*, and the like can be used. In this case, the promoter is not particularly limited as long as it can function in yeast, and examples include the *gal*1 promoter, *gal*10 promoter, heat shock protein promoter, MFα1 promoter, PHO5 promoter, PGK promoter, GAP promoter, ADH promoter, and AOX1 promoter.

Methods for introducing recombinant vectors into yeast are not particularly limited as long as they allow introduction of DNA into yeast, and examples include electroporation, spheroplast methods, and lithium acetate methods.

When animal cells are used as host cells, monkey cells COS-7, Vero, Chinese hamster ovary cells (CHO cells), mouse L cells, rat GH3 cells, human FL cells, and the like can be used. In this case, the promoter is not particularly limited as long as it can function in animal cells, and examples include the SRα promoter, SV40 promoter, LTR (Long Terminal Repeat) promoter, CMV promoter, and early gene promoters of human cytomegalovirus.

Methods for introducing recombinant vectors into animal cells are not particularly limited as long as they allow introduction of DNA into animal cells, and examples include electroporation, calcium phosphate methods, and lipofection.

When insect cells are used as host cells, ovarian cells of *Spodoptera frugiperda*, ovarian cells of *Trichoplusia ni*, and cultured cells derived from silkworm ovaries can be used. Examples include Sf9 and Sf21 as ovarian cells of *S. frugiperda*, High 5 and BTI-TN-5B1-4 (manufactured by Invitrogen) as ovarian cells of *Trichoplusia ni*, and Bombyx mori N4 as cultured cells derived from silkworm ovaries.

Methods for introducing a recombinant vector into insect cells are not particularly limited as long as DNA can be introduced into insect cells, and examples thereof include a calcium phosphate method, a lipofection method, and an electroporation method.

By culturing transformant cells into which a recombinant vector incorporating DNA encoding a protein of interest has been introduced, the protein is produced. Culturing of the transformant cells can be carried out according to conventional methods used for culturing host cells.

As a medium for culturing transformant cells obtained using microorganisms such as *Escherichia coli* and yeast as hosts, either a natural medium or a synthetic medium may be used, as long as it contains carbon sources, nitrogen sources, inorganic salts, and the like that can be assimilated by the microorganism and enables efficient culturing of the transformant cells.

Examples of carbon sources include carbohydrates such as glucose, fructose, sucrose, and starch, organic acids such as acetic acid and propionic acid, and alcohols such as ethanol and propanol. Examples of nitrogen sources include ammonia; ammonium salts of inorganic acids or organic acids such as ammonium chloride, ammonium sulfate, ammonium acetate, and ammonium phosphate; peptone; meat extract; yeast extract; corn steep liquor; and casein hydrolysate. Examples of inorganic salts include potassium dihydrogen phosphate, dipotassium hydrogen phosphate, magnesium phosphate, magnesium sulfate, sodium chloride, ferrous sulfate, manganese sulfate, copper sulfate, and calcium carbonate.

Culturing of transformant cells obtained using microorganisms such as *Escherichia coli* and yeast as hosts can be carried out, for example, under aerobic conditions such as shaking culture or aerated stirring culture. The culture temperature is usually 25 to 37°C, and the culture time is usually 12 to 48 hours, and during the culture period, the pH is usually maintained at 6 to 8. Adjustment of pH can be carried out using inorganic acids, organic acids, alkaline solutions, urea, calcium carbonate, ammonia, and the like. During culturing, antibiotics such as ampicillin and tetracycline may be added to the medium as necessary.

When culturing microorganisms transformed with an expression vector using an inducible promoter, an inducer may be added to the medium as necessary. For example, when culturing microorganisms transformed with an expression vector using a lac promoter, isopropyl-β-D-thiogalactopyranoside or the like may be added, and when culturing microorganisms transformed with an expression vector using a *trp* promoter, indole acrylic acid or the like may be added to the medium.

As a medium for culturing transformant cells obtained using animal cells as hosts, commonly used media such as RPMI 1640 medium, Eagle's MEM medium, DMEM medium, Ham F12 medium, Ham F12K medium, or media obtained by adding fetal bovine serum or the like thereto can be used. Culturing of transformant cells is usually carried out at 37°C for 3 to 10 days in the presence of 5% CO₂. During culturing, antibiotics such as kanamycin, penicillin, and streptomycin may be added to the medium as necessary.

As a medium for culturing transformant cells obtained using insect cells as hosts, commonly used media such as TNM-FH medium (manufactured by Pharmingen), Sf-900 II SFM medium (manufactured by Gibco BRL), ExCell 400, and ExCell 405 (manufactured by JRH Biosciences) can be used. Culturing of transformant cells is usually carried out at 27°C for 3 to 10 days. During culturing, antibiotics such as gentamicin may be added to the medium as necessary.

The protein of interest may be produced as a secreted protein or a fusion protein. Examples of proteins to be fused include β-galactosidase, protein A, an IgG-binding region of protein A, chloramphenicol acetyltransferase, poly(Arg), poly(Glu), protein G, maltosebinding protein, glutathione S-transferase, a polyhistidine chain (His-tag), S peptide, a DNA-binding protein domain, Tac antigen, thioredoxin, green fluorescent protein, and the like.

The protein of interest can be obtained by collecting the protein from a culture of transformant cells. Here, the term "culture" includes any of culture supernatant, cultured cells, cultured microbial cells, and disrupted products of cells or microbial cells.

When the protein of interest accumulates intracellularly in transformant cells, the cells in the culture are collected by centrifugation, washed, and then disrupted to extract the protein. When the protein is secreted extracellularly from the transformant cells, the culture supernatant can be used as it is, or cells or microbial cells can be removed from the culture supernatant by centrifugation or the like.

The obtained protein can be purified by extraction of cell lysate, salting-out using ammonium sulfate or the like, desalting, precipitation using organic solvents, diethylaminoethyl (DEAE)-Sepharose, ion-exchange chromatography, hydrophobic chromatography, gel filtration, affinity chromatography, and the like.

The protein of the present invention can also be produced by chemical synthesis methods such as the Fmoc method (fluorenylmethyloxycarbonyl method) and the tBoc method (t-butoxycarbonyl method) based on its amino acid sequence. In this case, a commercially available peptide synthesizer can be used.

The protein of the present invention forms a complex with crRNA (guide RNA), and the crRNA can guide the protein onto a target DNA strand. By utilizing this property, genome editing becomes possible. The present invention provides a method for genome editing using the protein of (a), (b), or (c) above and crRNA (guide RNA).

When the protein of the present invention has nuclease activity, it can cleave a target DNA strand guided by crRNA (guide RNA), thereby enabling modification of DNA. The present invention also provides a method for cleaving DNA having a sequence complementary to the spacer sequence in the crRNA using the protein of (a), (b), or (c) above and crRNA. In CRISPR-based gene targeting, target sequence-specific crRNA (guide RNA) and a nuclease (Cas protein) need to be co-expressed in a cell. The protein of the present invention and crRNA (guide RNA) may be expressed from a single vector, or may be co-expressed by expressing the protein of the present invention and crRNA (guide RNA) from separate vectors. Double-stranded DNA breaks induced by the protein of the present invention and crRNA (guide RNA) are repaired by either non-homologous end joining (NHEJ) or homology-directed repair (HDR). In NHEJ, errors frequently occur during repair due to repeated cleavage at the target site, resulting in insertions or deletions of bases, thereby enabling disruption of gene function (knockout). In HDR, repair is carried out using a homologous region of an uncut chromosome as a template. By utilizing this mechanism, replacement of a DNA sequence at a specific site on a chromosome or insertion of exogenous DNA at a specific site (knock-in) becomes possible. Specifically, a DNA fragment (donor DNA) in which the DNA to be replaced or inserted is flanked by sequences upstream and downstream of the target site on the chromosome is prepared. When this donor DNA is present at the site of a double-stranded DNA break, the donor DNA is inserted into the cleavage site (knock-in). Genome editing-based gene modification enables elucidation of gene functions, breeding improvement, treatment of diseases, and creation of disease model animals reproducing human genetic abnormalities.

When the protein of the present invention does not have nuclease activity, it can be used for transcriptional regulation (activation or repression) of specific genes. In addition, by labeling the protein of the present invention with a fluorescent dye, biotin, an enzyme, or the like, it can be used for site-specific imaging and nucleic acid detection methods. Furthermore, by linking the protein of the present invention to an enzyme that modifies the epigenome, site-specific epigenetic modification becomes possible.

The present invention also provides a kit for genome editing. The kit of the present invention comprises at least one selected from the group consisting of (i), (ii), and (iii) below:
(i) the protein of (a), (b), or (c) above;
(ii) mRNA encoding the protein of (a), (b), or (c) above; and
(iii) an expression vector containing DNA encoding the protein of (a), (b), or (c) above.

The protein of (a), (b), or (c) above may have nuclease activity or may lack nuclease activity, and may be labeled with a fluorescent dye, biotin, an enzyme, or the like.

The mRNA encoding the protein of (a), (b), or (c) above may be chemically modified with pseudouridine, 5-methylcytosine, 5-methoxyuridine, or the like, may be codonoptimized, and may include a cap structure, a poly(A) tail, and the like.

In the expression vector comprising DNA encoding the protein of (a), (b), or (c) above, viral vectors such as adenovirus and adeno-associated virus (AAV), or plasmid vectors can be used as the expression vector. The vector may contain, in addition to a promoter, cis-elements such as enhancers, splicing signals, polyA addition signals, selection markers (for example, a dihydrofolate reductase gene, an ampicillin resistance gene, and a neomycin resistance gene), ribosome-binding sequences (SD sequences), and the like. The expression vector may be linearized. The expression vector may include a repeat sequence of crRNA.

The kit of the present invention may further include a buffer, crRNA as a negative control, crRNA as a positive control, instructions for use, and the like.

### Examples

The present invention will be described in more detail by way of Examples below.

### [Example 1]

### Metagenomic Analysis

Seawater samples were collected from a fixed sampling point in Sendai Bay, Japan. The samples were fractionated based on cell size by sequential filtration through three filters with different pore sizes (8 µm, 1 µm, and 0.22 µm). DNA samples were prepared separately from the bacterial/archaeal fraction (retained on the 0.22 µm filter) and the viral/phage fraction (passed through the 0.22 µm filter). Each DNA sample was subjected to next-generation sequencing (NGS) using the Illumina MiSeq platform (Figure 1). The resulting metagenomic sequencing reads were assembled to generate contiguous sequence data as comprehensively as possible. The assembled metagenomic database was then screened for CRISPR-like repeat sequences, resulting in the identification of several putative CRISPR loci. Detailed sequence comparison of candidate Cas protein-coding regions located in the vicinity of the CRISPR arrays revealed an open reading frame (ORF) encoding a protein resembling a Type V Cas effector (Figure 2). To further verify the accuracy of the assembled sequence, the corresponding genomic region was amplified by PCR using the original environmental DNA as a template and re-sequenced by Sanger dideoxy sequencing, which provides higher accuracy than NGS.

To analyze the function of the polypeptide encoded by this gene, an expression plasmid was constructed by inserting the gene into the vector pET21a(+) (Merck) for expression in *E. coli.* This expression plasmid was then transformed into the *E. coli* host BL21(DE3 CodonPlus-RIL (Agilent) to produce the target polypeptide. However, purification was difficult due to instability. Therefore, a neighboring CRISPR-like sequence was also inserted into pETDuet1 (Merck) to construct the pETDuet1-6His-TD1+array for co-expression with the crRNA*. E. coli* BL21(DE3) CodonPlus-RIL was transformed with this expression plasmid and cultured. As a result, the production efficiency of the target polypeptide (designated TD1) increased, and TD1 was purified to homogeneity, as described below (Figure 3).

### Co-expression of the polypeptide encoded by the ORF and its surrounding region

The expression vector was designed to contain six histidines (His) at the amino terminus of the expressed polypeptide. 6His-TD1 produced in *E. coli* was first affinitypurified using Ni-NTA agarose (Qiagen). The polypeptide that specifically bound to Ni-NTA agarose was fractionated and further purified by heparin affinity chromatography (HiTrap Heparin HP, Cytiva). This fraction was concentrated by ultrafiltration and then loaded onto a Superdex 200 Increase 10/300 GL (Cytiva). The main peak was confirmed to contain 6His-TD1 (Figure 3). Furthermore, the UV absorption of the fraction eluted with the main peak was higher at 260 nm than at 280 nm (A₂₈₀ < A₂₆₀), suggesting that this fraction contained nucleic acids (Figure 3).

### Identification of Nucleic Acids Associated with TD1

To identify the nucleic acids contained in the purified fractions obtained by coexpressing the CRISPR region and TD1 protein, the components contained in the peak fractions obtained by gel filtration were separated into polypeptides and nucleic acids by phenol-chloroform-isoamyl alcohol (PCI) extraction, and the nucleic acids were recovered by ethanol precipitation. The recovered nucleic acids were then treated with DNase or RNase and analyzed by denaturing polyacrylamide gel electrophoresis (PAGE) (Figure 4). As a result, it was found that the nucleic acid contained in the complex was not degraded by DNase but was degraded by RNase, indicating that they were RNA molecules of approximately 50 nucleotides in length (Figure 4). The isolated RNAs were further analyzed by RNA sequencing (RNA-seq) using next-generation sequencing (NGS). Of all sequencing reads obtained, 98% mapped to the plasmid pETDuet1-6His-TD1+array, with a particularly high abundance of reads derived from the CRISPR array region (Figure 5).
The most abundant RNA species corresponded to sequences containing a portion of the CRISPR repeat and spacer regions, indicating that the RNA bound to TD1 was crRNA derived from the CRISPR array. These results suggest that TD1 itself processes precursor crRNA transcribed from the CRISPR region and cleaves it to generate crRNA of the appropriate length. Specifically, TD1 is presumed to recognize and bind to the stem-loop structure of the repeat sequence and cleave it at two positions: upstream of the stem-loop and within the spacer region.

### Specific cleavage activity of target DNA and identification of PAM

An experimental system was constructed to examine the target DNA cleavage reaction using the purified TD1-crRNA complex. The target DNA was the 5'-most spacer sequence in the CRISPR array. The DNA substrate was generated using pUC18. A short sequence called a Protospacer Adjacent Motif (PAM) is required for the Cas effector to recognize the target sequence, and the PAM sequence varies among different Cas effectors. To determine the PAM specificity of TD1, a PAM library containing all possible combinations of five nucleotides (5'-NNNNN-3') (in which each base in the five-base sequence was a mixture of A, G, C, and T) was inserted adjacent to the target sequence. The target sequence was a human DNMT1 (an enzyme that methylates cytosines in genomic DNA) gene fragment. When this plasmid was used as a substrate for cleavage by TD1, it was confirmed that the circular plasmid was cleaved and linearized. Using this reaction system, the PAM sequence preferred by TD1 and the cleavage site were determined.
Because it was unknown how double-stranded DNA cleavage occurred, the cleavage reaction product was first blunt-ended and a single A was added. T-overhang adapter was then ligated, and PCR amplification was performed using primers based on the adapter sequence. The amplified product was then sequenced using NGS to determine the sequence obtained at the PAM position. Specificity was observed, with a C at the second position from the 3' end of 5'-NNNNN-3' (Figure 6). Analysis of cleavage sites further indicated that TD1 primarily cleaves the phosphodiester bonds at positions 19 and 21 nucleotides downstream from the PAM, generating staggered double-strand breaks with 3-nucleotide 5' overhangs (Figure 7).

### PAM Sequence Specificity

To further investigate PAM specificity, a recognized PAM sequence (5'-GATCA-3') was used as a template, and each nucleotide position was individually substituted with all four nucleotides. Cleavage efficiency was compared among these variants. While substitutions at positions 1, 3, and 4 had minimal effects on cleavage efficiency, substitutions at position 2 significantly reduced cleavage activity unless cytosine was present (Figure 8), indicating that TD1 PAM recognition is primarily determined by a cytosine at the second position.

Based on this result, the PAM position was set to 5'-CCTCA-3', and cleavage reactions were conducted using 270 nM TD1-crRNA complex and 5.7 nM target DNA in rCutSmart buffer (50 mM potassium acetate, 20 mM Tris-acetate, 10 mM magnesium acetate, 100 µg/mL recombinant albumin, pH 7.9 at 25°C; New England Biolabs). After 20 minutes, much of the DNA remained uncleaved, but after 30 minutes of reaction, almost all of the DNA was cleaved. Therefore, we compared the results under these conditions by changing the reaction temperature. The cleavage reaction was carried out for 60 minutes. The results showed that the cleavage reaction proceeded efficiently between 37 and 45°C (Figure 9). At 4°C or above 50°C, almost no cleavage occurred, and at 20~30°C, many open circles (OCs), where only one strand was cleaved, were observed (Figure 9).

### Generation of TD1 mutants

To generate a catalytically inactive TD1 mutant, site-directed mutagenesis was performed based on predicted active-site residues. Among Asp308, Glu470, and Asp558, which were predicted to constitute the nuclease catalytic active center, Asp308 was substituted with alanine to generate a cleavage-deficient mutant (Figure 10).

The mutant TD1 protein was mixed with target DNA- crRNA shown in Figure 10 in the same manner as wild-type TD1 and subjected to gel filtration (size-exclusion chromatography). The peak fraction [1] containing the TD1 mutant, crRNA, and target DNA was subjected to cryo-electron microscopy, which provided good particle images for structural analysis. A total of 1,248,508 particles were selected and classified to obtain nine classes of two-dimensional averaged images (Figure 11). Based on this data, a three-dimensional structure model was constructed and a model structure was obtained at 2.8 Å resolution (Figure 11). This structure contained crRNA and target DNA, and captured the state in which crRNA penetrated into the double-stranded target DNA to form a hybrid strand (R-loop formation) (Figure 12). Furthermore, structural analysis identified amino acid residues that can recognize PAM recognition. When mutants were actually constructed and examined, it was confirmed that mutants in which Arg191 and Arg193 were substituted with alanine (R191A, R193A), respectively, exhibited almost no cleavage activity (Figure 13).

All publications, patents, and patent applications cited herein are hereby incorporated by reference in their entirety.

### Industrial Applicability

The present invention is applicable to genome editing technologies.

### Sequence Listing Free Text

SEQ ID NO: 1: Sequence of the gene region encoding the novel CRISPR-Cas 
CTTGCTTTCCACATGTCAACGCATGGTAAGCAGTTTCCTGTAGCAGGTCT
GATAGCACGATAAGCTGGATGCACACGGCAACCTTTTACCAACAGTTCTA
GAAGCTCTATTTTATGAAGTAATTTTTTCATGTTTCAGTCTCCTTCATTT
TTACTTGGGTAAAAACCTACCACAAAATCTATTCCAAACATCGTACTTGC
ACCAATACTCACAAGTCGTGTTTTCAACCATGATTTCCTCAAAGGGTTTA
CCTTCTATCGAGCAGTATCCAACCTTTCTGTCATATGTCGTTTTACCAGT
GAGTTCACAAATGGCTTGCTTGCCTTTGAACTGTTTTGCAAATCGTGTCG
CAGCTTTACCTTCGCTTGTACTGTTTTCAGGGCAATCCAAAACAGCAATA
CGAATAGGAACTCCTTTTACCTCAAACGTATCACCGTCACGAACATGAGT
TATAGTGCCTCGTAAAACTGATGCCGCTCTTTTATCAAACCCTGTTTCTG
GGTCTGTATATCCGATGCCTAGTTCATCCATATAGTTCAGAAACAAAAAC
GCAGAGGCAATAAGTATTACTACAATAAGGTTTGATGAAGGACTGAAGTA
GCGTCTTCTCCTTGGTCTAGCGTTGGATACAACCCTTACGCCAGTTCGCT
TTTTGAATGCCGCAGTCGTATCTTTCTTTTTGCCTGACTTCCTGACAGGT
CTTCTGTTCCAAGGTGACGGTGGGGGTTTCTTTGTCATATACTTAACTAG
TCTTTATTCTTCATCTCTTTCATAGCTTGATCAAGAGATGTAAACTTTAG
GTTCTCAGAACCACACTCGTCACATTTATTTGAGAGATCCATACCTTCTG
GCAGTGCCATCCAACTGTTATGACTCAGTAACTGCATCTTGAGTGGCATA
ACATGTCCACAGTCCAAACAATCAGCATGATATGGTTTAATTACGGTCAT
CTAAAACAATACCTAAAACCTTGTTACAAGATGATACAAACACCATCAGC
TATTGTTTTGGCAAGTATCCTACACCTTGATCCAGTTAAACTTTTAAGTA
TCATCTTGGTATGGCAACTCGAGTCTACAAATATGGTCTGATACCTATTG
GCTACCCTCCGCAGGAAGCTATTGATGAACTGTTTAGGGCTAATAAACTG
TGGAACACTCTTGTTGCTTTACACAAAGAGCATTTCGAGAGATGGGAAGA
TGCCAGACGTACGGCATCTATTGAATACTCAGAAAAGTTAGATGAACTAG
AGGTTAAACAACAACAGATAATGGATGCATTTGATGCCTTGCGACAAGTA
CGGATGGAAGAAGGCACTAAAGACGAGAGCAATCCAAGGCTAAAGTTGCA
AAGAGATGTTATTAACAGGCTCAGAGATGAACAGAGTGTTATATTTACAG
AGTTAAAGCCTCTTCGAAATGCAGCCAATAAACTGATAGATACAAAAGAG
ATAAATGACATATTTCGAACCAAGGTAAACGCTGCAGCTTCAACCAAAAC
TAATGGTCTCTATAATCCAACGGCTGATGAAGTTGTGAGAAACTTTAAAG
AAGCAAGACAACGTATCTTTGCCAATCCTAAGTCTCAGCTACGTTTCCAT
CGTTTTGATGGCACTGGCTACTACAACTTTAGATTTAGACGTAAAGGTGA
AAAACAGGATGGTGTTTGGTTCTCAGAGTTATTTGCTGGTAACGCAAAAG
AAGACAAACGGTTCTCAGTAATTGGTAGAGACGATAGCCACAAGAAGACA
CGTCTACGATTAAGAGCTACGATGACTGGTGGGGCAACCAAGGCTAGTAA
AGTATATCAAGAGTTTGATTGGATATTACATAGAGATATCTCAGAAAATG
CTCAGATCCAAAACGGTAAGATCCTAAGAACACGCTCTGGCGATAAGTTT
 
AAATATGATCTCGTTTTAACATTACGAATACCTGATACTGAAACTCTTCC
ACCAAACAGCCTTAAAGGCACTATTGGTATAGATGTTGGATTTAGGAAGT
CTGGTGATACCGTACTTGTTGCCACTGTTCTGTCAGATGAAGTTGGAGCC
AAACCATTGGAGATCAAAGCACCTTCAAAGATGGTATCTGCTCTGGAACA
CGTCATAGATTTACAGGGTGAACTAGACGATGCTGCTAACGACTTAGGTA
AAGCTATTACACCACTAATGAAGCAGCACCGTTTAGAAGAGGATCACCAA
AAGTATCGCTTTTGTAAGTCAATTACAGACAGACCTGCCAATGTGACCCT
GTCCTATGAAAAAGCGTATAAGCTAGCGATATGGCTAAACCACGAACCTG
ATGCTCTACCTAAAGAGATTACGGCTAAAGTCTTCACGTGGTGGCGCAGC
TACAGTCGCAAATATAGAGAGATACATAACAGAAGACGCAAGCAGCTTAC
GCACCGCAAGCACTTCTACAGAGAAGCTGCCGCCAGTATTGTTGCCCAAC
AAAAGTTAATTGTTTTGGAGAAGATTAATCTAGCACGTTTTGCTGAGACC
AAAGACAAAAACACTAAGCTCACCAATAAAGCCAGAGCGCAACGGTTCTT
AGGTTCAGTGTCAGAGTTAAGAGATGCAATAAAGAATGCAGCGGATAGAG
AAGGTGTTCCTTACATAGAGGTGAATCCTGCGTACACGTCTAAAACATGC
TCTGAGTGTGGTGCTTTAAATAAAGAGTTAAAGGCTGAGAAGGAGTGGAC
TTGTCAGTCATGTGGTGTCGTACATGACAGAGATACTAATGCTGCGAATA
ACTTACAAAACATGGGTAAGCAGTACTTAGATTATGTAAAAAACAAAGAT
GATGCGGTAATAGAGTAAGCAACAGATGCAGTTTTTAGCTAGTCCAAATC
GTCTTTTTGCTATAGATAACAAGGAGGTATTAACCTATCAGATTAAGGCC
CTTGTGTAGTGGGGTGTAACTACAACTTATAACTAAACCGCACGTTACAA
TACCAGGATTAAGGCCCTTGTGTAGTGGGGTGTAACTACAACTACCTGAT
AGAGTGTAAGAGGTATAAAGCAGATTAAGGCCCCTGTGTAGTGGTGTGTA
ACTACAACGAGGAATGACTTGACCTTGCGAGTCATAAGATTAAGGCCCCT
GTGTAGTGGGGTGTAACTACAACTATTGCTGTGCTTCCGTCATAATCTGT
TGTGATTAAGGCCCCTGTGTAGTGGGGTGTAACTACAACGAGGTTACTGA
TCTCGTCACGTTCTCTATCGATTAAGGCCCCTGTGCGTTGGGGTGTAAAC
GCAACCCGTGGGTGTTAACAGCGTTAAAACCTTTGAGTTACGGCCCTTGT
GTAGTGGGGTGTAAACAAATAGGTGCTTAGTCATAAAGGCTAGGCATCTT
TTGTTTAAGCTATCCAGTTAAAAAACCACATCTGTGTAAAGTGATATATA
CCACTACGGATGCCATGCCAGTGGCCCTCCAACACACCTCTAAAACTTTT
TGATGTTCAGGTTCTAGGGCAATCAGTATCTACAAATAGAGAGTACCAAT
AGAGCAAGGTCTAAGAGGACTATACTATATAGTACCAATAGGGTTGTGTT
TTATATTTTTGGTACTATACTCCCACAATGTCTAAGACGTTAAAGGGAAT
ATAGATATGTTAATAGGTGTAGCTAGGACATCCACCATAGAACAAGTTGC
AGGGTTGGATGCACAAGTACAACAATTAAAAGAATATGGCTGTGAAAAGA
TTTTCCAAGAGCAAGTATCAAGCGTTGGTGAGCGTGAACAATTAAACGTA
G
 (The TD1 coding region is single-underlined, and the CRISPR repeat sequence is doubleunderlined.)
SEQ ID NO: 2: Nucleotide sequence of the TD1 coding region (1758 nucleotides) ATGGCAACTCGAGTCTACAAATATGGTCTGATACCTATTGGCTACCCTCCGCAGGA 
AGCTATTGATGAACTGTTTAGGGCTAATAAACTGTGGAACACTCTTGTTGCTTTACA
CAAAGAGCATTTCGAGAGATGGGAAGATGCCAGACGTACGGCATCTATTGAATACT
CAGAAAAGTTAGATGAACTAGAGGTTAAACAACAACAGATAATGGATGCATTTGAT
GCCTTGCGACAAGTACGGATGGAAGAAGGCACTAAAGACGAGAGCAATCCAAGG
CTAAAGTTGCAAAGAGATGTTATTAACAGGCTCAGAGATGAACAGAGTGTTATATT
TACAGAGTTAAAGCCTCTTCGAAATGCAGCCAATAAACTGATAGATACAAAAGAG
ATAAATGACATATTTCGAACCAAGGTAAACGCTGCAGCTTCAACCAAAACTAATGG
TCTCTATAATCCAACGGCTGATGAAGTTGTGAGAAACTTTAAAGAAGCAAGACAA
CGTATCTTTGCCAATCCTAAGTCTCAGCTACGTTTCCATCGTTTTGATGGCACTGGC
TACTACAACTTTAGATTTAGACGTAAAGGTGAAAAACAGGATGGTGTTTGGTTCTC
AGAGTTATTTGCTGGTAACGCAAAAGAAGACAAACGGTTCTCAGTAATTGGTAGA
GACGATAGCCACAAGAAGACACGTCTACGATTAAGAGCTACGATGACTGGTGGGG
CAACCAAGGCTAGTAAAGTATATCAAGAGTTTGATTGGATATTACATAGAGATATCT
CAGAAAATGCTCAGATCCAAAACGGTAAGATCCTAAGAACACGCTCTGGCGATAA
GTTTAAATATGATCTCGTTTTAACATTACGAATACCTGATACTGAAACTCTTCCACC
AAACAGCCTTAAAGGCACTATTGGTATAGATGTTGGATTTAGGAAGTCTGGTGATA
CCGTACTTGTTGCCACTGTTCTGTCAGATGAAGTTGGAGCCAAACCATTGGAGATC
AAAGCACCTTCAAAGATGGTATCTGCTCTGGAACACGTCATAGATTTACAGGGTGA
ACTAGACGATGCTGCTAACGACTTAGGTAAAGCTATTACACCACTAATGAAGCAGC
ACCGTTTAGAAGAGGATCACCAAAAGTATCGCTTTTGTAAGTCAATTACAGACAGA
CCTGCCAATGTGACCCTGTCCTATGAAAAAGCGTATAAGCTAGCGATATGGCTAAA
CCACGAACCTGATGCTCTACCTAAAGAGATTACGGCTAAAGTCTTCACGTGGTGGC
GCAGCTACAGTCGCAAATATAGAGAGATACATAACAGAAGACGCAAGCAGCTTAC
GCACCGCAAGCACTTCTACAGAGAAGCTGCCGCCAGTATTGTTGCCCAACAAAAG
TTAATTGTTTTGGAGAAGATTAATCTAGCACGTTTTGCTGAGACCAAAGACAAAAA
CACTAAGCTCACCAATAAAGCCAGAGCGCAACGGTTCTTAGGTTCAGTGTCAGAG
TTAAGAGATGCAATAAAGAATGCAGCGGATAGAGAAGGTGTTCCTTACATAGAGGT
GAATCCTGCGTACACGTCTAAAACATGCTCTGAGTGTGGTGCTTTAAATAAAGAGT
TAAAGGCTGAGAAGGAGTGGACTTGTCAGTCATGTGGTGTCGTACATGACAGAGA
TACTAATGCTGCGAATAACTTACAAAACATGGGTAAGCAGTACTTAGATTATGTAAA
AAACAAAGATGATGCGGTAATAGAGTAA
SEQ ID NO: 3: Amino acid sequence of TD1 (sequence translated in the single-underlined reading frame of SEQ ID NO: 1) 
MATRVYKYGLIPIGYPPQEAIDELFRANKLWNTLVALHKEHFERWEDARRTASIEYSE
KLDELEVKQQQIMDAFDALRQVRMEEGTKDESNPRLKLQRDVINRLRDEQSVIFTEL
KPLRNAANKLIDTKEINDIFRTKVNAAASTKTNGLYNPTADEVVRNFKEARQRIFANP
KSQLRFHRFDGTGYYNFRFRRKGEKQDGVWFSELFAGNAKEDKRFSVIGRDDSHKK
 
TRLRLRATMTGGATKASKVYQEFDWILHRDISENAQIQNGKILRTRSGDKFKYDLVLT
LRIPDTETLPPNSLKGTIGIDVGFRKSGDTVLVATVLSDEVGAKPLEIKAPSKMVSALE
HVIDLQGELDDAANDLGKAITPLMKQHRLEEDHQKYRFCKSITDRPANVTLSYEKAY
KLAIWLNHEPDALPKEITAKVFTWWRSYSRKYREIHNRRRKQLTHRKHFYREAAASI
VAQQKLIVLEKINLARFAETKDKNTKLTNKARAQRFLGSVSELRDAIKNAADREGVP
YIEVNPAYTSKTCSECGALNKELKAEKEWTCQSCGVVHDRDTNAANNLQNMGKQY
LDYVKNKDDAVIE
   Number of amino acids: 585
   Molecular weight: 67475.71
   Calculated pI: 9.42
SEQ ID NO: 4: crRNA repeat sequence (sequence obtained by excluding the 4 bases (GATT) on the 5' side from the CRISPR repeat sequence (the first or second double underlined sequence) in the sequence of SEQ ID NO: 1)
   AAGGCCCTTGTGTAGTGGGGTGTAACTACAAC
SEQ ID NO: 5: crRNA repeat sequence (sequence obtained by excluding the 4 bases (GATT) on the 5' side from the CRISPR repeat sequence (the third double underlined sequence) in the sequence of SEQ ID NO: 1)
   AAGGCC**CC**TGTGTAGTGG**T**GTGTAACTACAAC
SEQ ID NO: 6: crRNA repeat sequence (sequence obtained by excluding the 4 bases (GATT) on the 5' side from the CRISPR repeat sequence (the 4th and 5th double underlined sequence) in the sequence of SEQ ID NO: 1)
   AAGGCCC**C**TGTGTAGTGGGGTGTAACTACAAC
SEQ ID NO: 7: crRNA repeat sequence (sequence obtained by excluding the 4 bases (GATT) on the 5' side from the CRISPR repeat sequence (the 6th double underlined sequence) in the sequence of SEQ ID NO: 1)
   AAGGCCC**C**TGTG**CGT**TGGGGTGTAA**ACG**CAAC
SEQ ID NO: 8: crRNA repeat sequence (sequence obtained by excluding the 4 bases (GATT) on the 5' side from the CRISPR repeat sequence (the 7th double underlined sequence) in the sequence of SEQ ID NO: 1)
   A**C**GGCCCTTGTGTAGTGGGGTGTAA**AC**A**A**A**T**A

## Claims

1. A protein selected from the group consisting of:
(a) a protein comprising the amino acid sequence set forth in SEQ ID NO: 3;
(b) a protein comprising an amino acid sequence having at least 62% and less than 100% sequence identity to the amino acid sequence set forth in SEQ ID NO: 3, the protein being capable of forming a complex with a crRNA and binding to DNA having a sequence complementary to a spacer sequence in the crRNA; and
(c) a protein comprising an amino acid sequence in which 1 to 20 amino acids in the amino acid sequence set forth in SEQ ID NO: 3 are mutated, the protein being capable of forming a complex with a crRNA and binding to DNA having a sequence complementary to a spacer sequence in the crRNA.

2. The protein according to claim 1, wherein the protein has nuclease activity.

3. The protein according to claim 1, wherein the protein lacks nuclease activity.

4. A nucleic acid encoding the protein according to claim 1.

5. An expression vector comprising a DNA encoding the protein according to claim 1.

6. A cell transformed with the expression vector according to claim 5.

7. A method for producing a protein selected from the group consisting of (a), (b), and (c), comprising culturing the cell according to claim 6:
(a) a protein comprising the amino acid sequence set forth in SEQ ID NO: 3;
(b) a protein comprising an amino acid sequence having at least 62% and less than 100% sequence identity to the amino acid sequence set forth in SEQ ID NO: 3, the protein being capable of forming a complex with a crRNA and binding to DNA having a sequence complementary to a spacer sequence in the crRNA; and
(c) a protein comprising an amino acid sequence in which 1 to 20 amino acids in the amino acid sequence set forth in SEQ ID NO: 3 are mutated, the protein being capable of forming a complex with a crRNA and binding to DNA having a sequence complementary to a spacer sequence in the crRNA.

8. A method for cleaving DNA having a sequence complementary to a spacer sequence in a crRNA, comprising using the protein according to claim 1 and the crRNA.

9. A method for genome editing, comprising using the protein according to claim 1 and a crRNA.

10. A kit for genome editing, comprising at least one selected from the group consisting of the protein according to claim 1, an mRNA encoding the protein according to claim 1 and an expression vector comprising a DNA encoding the protein according to claim 1.

11. A crRNA comprising a repeat sequence comprising any one of the nucleotide sequences set forth in SEQ ID NOs: 4 to 8.

12. The protein according to claim 1,
wherein the protein comprising an amino acid sequence homologous to the amino acid sequence set forth in SEQ ID NO: 3, and
wherein acidic or basic amino acid residues at positions corresponding to Asp156, Arg191,
and Arg193 in the amino acid sequence set forth in SEQ ID NO: 3 form hydrogen bonds with bases in a PAM sequence of a target DNA.

13. The crRNA according to claim 11, wherein a spacer sequence forms an R-loop with a target DNA.
